# EUROPEAN PATENT APPLICATION

(11) **EP 2 322 554 A1**
(43) Date of publication of application: **18.05.2011**
(21) Application number: 10009611.4
(22) Date of filing: 29.06.2005
(51) Int. Cl.: C07K 16/24, A61K 39/395, A61P 19/02

(54) **Composition comprising an anti-TNF-alpha domain antibody for the treatment of rheumatoid arthritis**

(30) Priority: 30.06.2004 GB 0402829; 24.08.2004 US 925366; 04.04.2005 US 98758
(62) Divisional of application: 05755389.3
(71) Applicant: Domantis Limited, Cambridge Cambridgeshire, CB4 OWG (GB)
(72) Inventor: Woolven, Benjamin, Cambridge CB4 0WG (GB); Tomlinson, Ian M., Cambridge CB4 0WG (GB); Jennings, Philip Anthony, Warrawee New South Wales 2074 (AU); Doyle, Anthony Gerard, New South Wales 2047 (AU)
(74) Representative: Lock, Graham James

(57) **Abstract**

The invention relates to compositions and methods for treating inflammatory disorders. More specifically, the invention relates to antibody compositions and their use in the treatment of inflammatory disorders.

## Description

This application is a divisional of European Application no. 1761565, the disclosure of which is included herein in its entirety.

The present invention relates to methods of treatment of diseases, including rheumatoid arthritis using antibody polypeptide constructs including single domain antibody ligands, compositions of such ligands, and methods of making and using such ligands. In particular, the invention provides methods for the preparation of single domain antibodies that bind TNF-α.

### TNF-α:

As the name implies, Tumor Necrosis Factor-a (TNF-α) was originally described as a molecule having anti-tumor properties, but the molecule was subsequently found to play key roles in other processes, including a prominent role in mediating inflammation and autoimmune disorders. TNF-α is a key proinflammatory cytokine in inflammatory conditions including, for example, rheumatoid arthritis (RA), Crohn's disease, ulcerative colitis and other bowel disorders, psoriasis, toxic shock, graft versus host disease and multiple sclerosis.

The pro-inflammatory actions of TNF-α result in tissue injury, such as inducing procoagulant activity on vascular endothelial cells (Pober, et al., J. Immunol. 136:1680 (1986)), increasing the adherence of neutrophils and lymphocytes (Pober, et al., J. Immunol. 138:3319 (1987)), and stimulating the release of platelet activating factor from macrophages, neutrophils and vascular endothelial cells (Camussi, et al., J. Exp. Med. 166:1390 (1987)).

TNF-α is synthesized as a 26 kD transmembrane precursor protein with an intracellular tail that is cleaved by a TNF-α-converting metalloproteinase enzyme and then secreted as a 17 kD soluble protein. The active form consists of a homotrimer of the 17 kD monomers which interacts with two different cell surface receptors, p55 TNFR1 and p75 TNFR2. There is also evidence that the cell surface bound precursor form of TNF-α can mediate some biological effects of the factor. Most cells express both p55 and p75 receptors which mediate different biological functions of the ligand. The p75 receptor is implicated in triggering lymphocyte proliferation, and the p55 receptor is implicated in TNF-mediated cytotoxicity, apoptosis, antiviral activity, fibroblast proliferation and NF-κB activation (see Locksley et al., 2001, Cell 104: 487-501).

The TNF receptors are members of a family of membrane proteins including the NGF receptor, Fas antigen, CD27, CD30, CD40, Ox40 and the receptor for the lymphotoxin α/β heterodimer. Binding of receptor by the homotrimer induces aggregation of receptors into small clusters of two or three molecules of either p55 or p75. TNF-α is produced primarily by activated macrophages and T lymphocytes, but also by neutrophils, endothelial cells, keratinocytes and fibroblasts during acute inflammatory reactions.

TNF-α is at the apex of the cascade of pro-inflammatory cytokines (Reviewed in Feldmann & Maini, 2001, Ann. Rev. Immunol. 19:163). This cytokine induces the expression or release of additional proinflammatory cytokines, particularly IL-1 and IL-6 (see, for example, Rutgeerts et al., 2004, Gastroenterology 126: 1593-1610). Inhibition of TNF-α inhibits the production of inflammatory cytokines including IL-1, IL-6, IL-8 and GM-CSF (Brennan et al., 1989, Lancet 2: 244).

Because of its role in inflammation, TNF-α has emerged as an important inhibition target in efforts to reduce the symptoms of inflammatory disorders. Various approaches to inhibition of TNF-α for the clinical treatment of disease have been pursued, including particularly the use of soluble TNF-α receptors and antibodies specific for TNF-α. Commercial products approved for clinical use include, for example, the antibody products Remicade^{™} (Infliximab; Centocor, Malvern, PA; a chimeric monoclonal IgG antibody bearing human IgG4 constant and mouse variable regions), Humira^{™} (adalimumab or D2F7; Abbott Laboratories, described in U.S. patent No. 6,090,382) and the soluble receptor product Enbrel^{™} (etanercept, a soluble p75 TNFR2 Fc fusion protein; Immunex).

The role of TNF-α in inflammatory arthritis is reviewed in, for example, Li & Schwartz, 2003, Sringer Semin. Immunopathol. 25: 19-33. In RA, TNF-α is highly expressed in inflamed synovium, particularly at the cartilage-pannus junction (DiGiovine e tal., 1988, Ann. Rheum. Dis. 47: 768; Firestein et al., 1990, J. Immunol. 144: 3347; and Saxne et al., 1988, Atrhritis Rheum. 31: 1041). In addition to evidence that TNF-α increases the levels of inflammatory cytokines IL-1, IL-6, IL-8 and GM-CSF, TNF-α can alone trigger joint inflammation and proliferation of fibroblast-like synoviocytes (Gitter et al., 1989, Immunology 66: 196), induce collagenase, thereby triggering cartilage destruction (Dayer et al., 1985, J. Exp. Med. 162: 2163; Dayer et al., 1986, J. Clin. Invest. 77: 645), inhibit proteoglycan synthesis by articular chondrocytes (Saklatvala, 1986, Nature 322: 547; Saklatvala et al., 1985, J. Exp. Med. 162: 1208) and can stimulate osteoclastogenesis and bone resorption (Abu-Amer et al., 2000, J. Biol. Chem. 275: 27307; Bertolini et al., 1986, Nature 319: 516). TNF-α induces increased release of CD14+ monocytes by the bone marrow. Such monocytes can infiltrate joints and amplify the inflammatory response via the RANK (Receptor Activator or NF-κB)-RANKL signaling pathway, giving rise to osteoclast formation during arthritic inflammation (reviewed in Anandarajah & Richlin, 2004, Curr. Opin. Rheumatol. 16: 338-343).

TNF-α is an acute phase protein which increases vascular permeability through its induction of IL-8, thereby recruiting macrophage and neutrophils to a site of infection. Once present, activated macrophages continue to produce TNF-α, thereby maintaining and amplifying the inflammatory response.

Titration of TNF-α by the soluble receptor construct etanercept is effective for the treatment of RA, but not for treatment of Crohn's disease. In contrast, the antibody TNF-α antagonist infliximab is effective to treat both RA and Crohn's disease. Thus, the mere neutralization of soluble TNF-α is not the only mechanism involved in anti-TNF-based therapeutic efficacy. Rather, the blockade of other pro-inflammatory signals or molecules that are induced by TNF-α also plays a role (Rutgeerts et al., supra). For example, the administration of infliximab apparently decreases the expression of adhesion molecules, resulting in a decreased infiltration of neutrophils to sites of inflammation. Also, infliximab therapy results in the disappearance of inflammatory cells from previously inflamed bowel mucosa in Crohn's disease. This disappearance of activated T cells in the lamina propria is mediated by apoptosis of cells carrying membrane-bound TNF-α following activation of caspases 8, 9 and then 3 in a Fas dependent manner (see Lugering et al., 2001, Gastroenterology 121:1145-1157). Thus, membrane- or receptor-bound TNF-α is an important target for anti-TNF-α therapeutic approaches. Others have shown that infliximab binds to activated peripheral blood cells and lamina propria cells and induces apoptosis through activation of caspase 3 (see Van den Brande et al., 2003, Gastroenterology 124: 1774-1785).

Intracellularly, the binding of trimeric TNF-α to its receptor triggers a cascade of signaling events, including displacement of inhibitory molecules such as SODD (silencer of death domains) and binding of the adaptor factors FADD, TRADD, TRAF2, c-IAP, RAIDD and TRIP plus the kinase RIP1 and certain caspases (reviewed by Chen & Goeddel, 2002, Science 296: 1634-1635, and by Muzio & Saccani in :Methods in Molecular Medicine: Tumor Necrosis Factor, Methods and Protocols," Corti and Ghezzi, eds. (Humana Press, New Jersey), pp. 81-99. The assembled signaling complex can activate either a cell survival pathway, through NF-κB activation and subsequent downstream gene activation, or an apoptotic pathway through caspase activation.

Similar extracellular downstream cytokine cascades and intracellular signal transduction pathways can be induced by TNF-α in other diseases. Thus, for other diseases or disorders in which the TNF-α molecule contributes to the pathology, inhibition of TNF-α presents an approach to treatment.

### Antibody Polypeptides:

Antibodies are highly specific for their binding targets and although they are derived from nature's own defense mechanisms, antibodies face several challenges when applied to the treatment of disease in human patients. Conventional antibodies are large multi-subunit protein molecules comprising at least four polypeptide chains. For example, human IgG has two heavy chains and two light chains that are disulfide bonded to form the functional antibody. The size of a conventional IgG is about 150 kD. Because of their relatively large size, complete antibodies (e.g., IgG, IgA, IgM, etc.) are limited in their therapeutic usefulness due to problems in, for example, tissue penetration. Considerable efforts have focused on identifying and producing smaller antibody fragments that retain antigen binding function and solubility.

The heavy and light polypeptide chains of antibodies comprise variable (V) regions that directly participate in antigen interactions, and constant (C) regions that provide structural support and function in non-antigen-specific interactions with immune effectors. The antigen binding domain of a conventional antibody is comprised of two separate domains: a heavy chain variable domain (V_{H}) and a light chain variable domain (V_{L}: which can be either V_{κ} or V_{λ}). The antigen binding site itself is formed by six polypeptide loops: three from the V_{H} domain (H1, H2 and H3) and three from the V_{L} domain (L1, L2 and L3). *In vivo,* a diverse primary repertoire of V genes that encode the V_{H} and V_{L} domains is produced by the combinatorial rearrangement of gene segments. C regions include the light chain C regions (referred to as C_{L} regions) and the heavy chain C regions (referred to as C_{H}1, C_{H}2 and C_{H}3 regions).

A number of smaller antigen binding fragments of naturally occurring antibodies have been identified following protease digestion. These include, for example, the "Fab fragment" (V_{L}-C_{L}-C_{H}l-V_{H}), "Fab' fragment" (a Fab with the heavy chain hinge region) and "F(ab')₂ fragment" (a dimer of Fab' fragments joined by the heavy chain hinge region). Recombinant methods have been used to generate even smaller antigen-binding fragments, referred to as "single chain Fv" (variable fragment) or "scFv," consisting of V_{L} and V_{H} joined by a synthetic peptide linker.

### Single Domain Antibodies:

While the antigen binding unit of a naturally-occurring antibody (e.g., in humans and most other mammals) is generally known to be comprised of a pair of V regions (V_{L}/V_{H}), camelid species express a large proportion of fully functional, highly specific antibodies that are devoid of light chain sequences. The camelid heavy chain antibodies are found as homodimers of a single heavy chain, dimerized via their constant regions. The variable domains of these camelid heavy chain antibodies are referred to as V_{H}H domains and retain the ability, when isolated as fragments of the V_{H} chain, to bind antigen with high specificity ((Hamers-Casterman et al., 1993, Nature 363:446-448; Gahroudi et al., 1997, FEBS Lett. 414: 521-526). Antigen binding single V_{H} domains have also been identified from, for example, a library of murine V_{H} genes amplified from genomic DNA from the spleens of immunized mice and expressed in *E. coli* (Ward et al., 1989, Nature 341: 544-546). Ward et al. named the isolated single V_{H} domains "dAbs," for "domain antibodies." The term "dAb" will refer herein to a single immunoglobulin variable domain (V_{H}, V_{HH} or V_{L}) polypeptide that specifically binds antigen. A "dAb" binds antigen independently of other V domains; however, as the term is used herein, a "dAb" can be present in a homo- or heteromultimer with other V_{H} or V_{L} domains where the other domains are not required for antigen binding by the dAb, i.e., where the dAb binds antigen independently of the additional V_{H}, V_{HH} or V_{L} domains.

Single immunoglobulin variable domains, for example, V_{H}H, are the smallest antigen-binding antibody unit known. For use in therapy, human antibodies are preferred, primarily because they are not as likely to provoke an immune response when administered to a patient. As noted above, isolated non-camelid V_{H} domains tend to be relatively insoluble and are often poorly expressed. Comparisons of camelid V_{H}H with the V_{H} domains of human antibodies reveals several key differences in the framework regions of the camelid V_{H}H domain corresponding to the V_{H}/V_{L} interface of the human V_{H} domains. Mutation of these residues of human V_{H}3 to more closely resemble the V_{H}H sequence (specifically Gly 44→Glu, Leu 45→Arg and Trp 47→Gly) has been performed to produce "camelized" human V_{H} domains that retain antigen binding activity (Davies & Riechmann, 1994, FEBS Lett. 339: 285-290) yet have improved expression and solubility. (Variable domain amino acid numbering used herein is consistent with the Kabat numbering convention (Kabat et al., 1991, Sequences of Immunological Interest, 5th ed. U.S. Dept. Health & Human Services, Washington, D.C.)) WO 03/035694 (Muyldermans) reports that the Trp 103→Arg mutation improves the solubility of non-camelid V_{H} domains. Davies & Riechmann (1995, Biotechnology N.Y. 13: 475-479) also report production of a phage-displayed repertoire of camelized human V_{H} domains and selection of clones that bind hapten with affinities in the range of 100-400 nM, but clones selected for binding to protein antigen had weaker affinities.

The antigen binding domain of an antibody comprises two separate regions: a heavy chain variable domain (V_{H}) and a light chain variable domain (V_{L}: which can be either V_{κ} or V_{λ}). The antigen binding site itself is formed by six polypeptide loops: three from V_{H} domain (H1, H2 and H3) and three from V_{L} domain (L1, L2 and L3). A diverse primary repertoire of V genes that encode the V_{H} and V_{L} domains is produced by the combinatorial rearrangement of gene segments. The V_{H} gene is produced by the recombination of three gene segments, V_{H}, D and J_{H}. In humans, there are approximately 51 functional V_{H} segments (Cook and Tomlinson (1995) Immunol Today, 16: 237), 25 functional D segments (Corbett et al. (1997) J. Mol. Biol., 268: 69) and 6 functional J_{H} segments (Ravetch et al. (1981) Cell, 27: 583), depending on the haplotype. The V_{H} segment encodes the region of the polypeptide chain which forms the first and second antigen binding loops of the V_{H} domain (H1 and H2), whilst the V_{H}, D and J_{H} segments combine to form the third antigen binding loop of the VH domain (H3). The V_{L} gene is produced by the recombination of only two gene segments, V_{L} and J_{L}. In humans, there are approximately 40 functional V_{κ} segments (Schable and Zachau (1993) Biol. Chem. Hoppe- Seyler, 374: 1001), 31 functional V_{λ} segments (Williams et al. (1996) J. Mol. so Biol., 264: 220; Kawasaki et al. (1997) Genome Res., 7: 250), 5 functional J_{κ} segments (Hieter et al. (1982) J. Biol. Chef,., 257: 1516) and 4 functional J_{λ} segments (Vasicek and Leder (1990) J. Exp. Med., 172: 609), depending on the haplotype. The V_{L} segment encodes the region of the polypeptide chain which forms the first and second antigen binding loops of the V_{L} domain (L1 and L2), whilst the V_{L} and J_{L} segments combine to form the third antigen binding loop of the V_{L} domain (L3). Antibodies selected from this primary repertoire are believed to be sufficiently diverse to bind almost all antigens with at least moderate affinity. High affinity antibodies are produced by "affinity maturation" of the rearranged genes, in which point mutations are generated and selected by the immune system on the basis of improved binding.

Analysis of the structures and sequences of antibodies has shown that five of the six antigen binding loops (H1, H2, L1, L2, L3) possess a limited number of main-chain conformations or canonical structures (Chothia and Lesk (1987) d: Mol. Biol., 196: 901; Chothia et al. (1989) Nature, 342: 877). The main-chain conformations are determined by (i) the length of the antigen binding loop, and (ii) particular residues, or types of residue, at certain key position in the antigen binding loop and the antibody framework. Analysis of the loop lengths and key residues has enabled us to the predict the main-chain conformations of H1, H2, L1, L2 and L3 encoded by the majority of human antibody sequences (Chothia et al. (1992) J. Mol. Biol., 227: 799; Tomlinson et al. (1995) EMBO J., 14: 4628; Williams et al. (1996) J. Mol. Biol., 264: 220). Although the H3 region is much more diverse in terms of sequence, length and structure (due to the use of D segments), it also forms a limited number of main- chain conformations for short loop lengths which depend on the length and the presence of particular residues, or types of residue, at key positions in the loop and the antibody framework (Martin et al. (1996) J. Mol. Biol., 263: 800; Shirai et al. (1996) FEBS Letters, 399: 1.

### SUMMARY OF THE INVENTION

The present invention describes methods of treating a TNF-α-related inflammatory disorder in an individual suffering from such a disorder. The method comprises administering a therapeutically effective amount of a single domain antibody polypeptide construct to such an individual, wherein the single domain antibody polypeptide construct binds human TNF-α, and whereby the TNF-α-related disorder is treated.

In one aspect, the inflammatory disorder is rheumatoid arthritis, and the method comprises the use of one or more single domain antibody polypeptide constructs, wherein one or more of the constructs antagonizes human TNFα's binding to a receptor. The present invention describes compositions comprising one or more single domain antibody polypeptide constructs that antagonize human TNFα's binding to a receptor.

Also encompassed herein is the use of a polypeptide construct as described herein in the preparation of a medicament for the treatment of disease, particularly in the preparation of a medicament for the treatment of rheumatoid arthritis.

In one aspect, the invention encompasses a method of treating rheumatoid arthritis, the method comprising administering to an individual in need thereof a therapeutically effective amount of a composition comprising a single domain antibody polypeptide construct that antagonizes human TNFα's binding to a receptor, whereby the rheumatoid arthritis is treated.

In one embodiment, the composition prevents an increase in arthritic score when administered to a mouse of the Tg197 transgenic mouse model of arthritis.

In another embodiment, the single domain antibody polypeptide construct antagonizes human TNFα as measured in a standard L929 cytotoxicity cell assay.

The invention further encompasses a method of treating rheumatoid arthritis, the method comprising administering to an individual in need thereof a therapeutically effective amount of a composition comprising a single domain antibody polypeptide construct that antagonizes human TNFα's binding to a receptor, wherein the single domain antibody polypeptide construct inhibits the binding of human TNFα to a TNFα receptor, and whereby the rheumatoid arthritis is treated.

In one embodiment, the single domain antibody polypeptide construct specifically binds to human TNF-α which is bound to a cell surface receptor.

In another embodiment, the single domain antibody polypeptide construct has an in vivo tα-half life in the range of 15 minutes to 12 hours, In another embodiment, the single domain antibody polypeptide construct has an in vivo tβ-half life in the range of 1 to 6 hours. In another embodiment, the single domain antibody polypeptide construct has an in vivo tβ-half life in the range of 2 to 5 hours. In another embodiment, the single domain antibody polypeptide construct has an in vivo tβ-half life in the range of 3 to 4 hours. In another embodiment, the single domain antibody polypeptide construct has an in vivo tβ-half life in the range of 12 to 60 hours. In another embodiment, the single domain antibody polypeptide construct has an in vivo tβ-half life in the range of 12 to 48 hours. In another embodiment, the single domain antibody polypeptide construct has an in vivo tβ-half life in the range of 12 to 26 hours.

In another embodiment, the single domain antibody polypeptide construct has an in vivo AUC half-life value of 15 mg.min/ml to 150 mg.min/ml. In another embodiment, the single domain antibody polypeptide construct has an in vivo AUC half-life value of 15 mg.min/ml to 100 mg.min/ml. In another embodiment, the single domain antibody polypeptide construct has an in vivo AUC half-life value of 15 mg.min/ml to 75 mg.min/ml. In another embodiment, the single domain antibody polypeptide construct has an in vivo AUC half-life value of 15 mg.min/ml to 50 mg.min/ml.

In another embodiment, the single domain antibody polypeptide construct is linked to a PEG molecule. In another embodiment, the PEG-linked single domain antibody polypeptide construct has a hydrodynamic size of at least 24 kDa, and wherein the total PEG size is from 20 to 60 kDa. In another embodiment, the PEG-linked single domain antibody polypeptide construct has a hydrodynamic size of at least 200 kDa and a total PEG size of from 20 to 60 kDa. In another embodiment, the PEGylated proteins of the invention may be linked, on average, to 1, 2, 3, 4, 5, 6, 7, 8, 9, 10, 12, 15, 17, 20, or more polyethylene glycol molecules.

In another embodiment, the antibody construct comprises two or more single immunoglobulin variable domain polypeptides that bind human TNFα. In another embodiment, the antibody construct comprises a homodimer of a single immunoglobulin variable domain polypeptide that binds human TNFα. In another embodiment, the antibody construct comprises a homotrimer of a single immunoglobulin variable domain polypeptide that binds human TNFα. In another embodiment, the antibody construct comprises a homotetramer of a single immunoglobulin variable domain polypeptide that binds human TNFα.

In another embodiment, the single domain antibody polypeptide construct comprises the amino acid sequence of the antibody polypeptide TAR1-5-490.

The invention further encompasses a method of treating rheumatoid arthritis, the method comprising administering to an individual in need thereof, a therapeutically effective amount of a composition comprising a single domain antibody polypeptide construct that antagonizes human TNFα's binding to a receptor, wherein the composition prevents an increase in arthritic score when administered to a mouse of the Tg197 transgenic mouse model of arthritis, wherein the single domain antibody polypeptides construct binds human TNFα with a Kd of < 100 nM, wherein the single domain antibody polypeptide construct neutralizes human TNFα as measured in a standard L929 cell assay, and wherein the rheumatoid arthritis is treated.

The invention further encompasses a composition comprising a single domain antibody polypeptide construct that antagonizes human TNFα's binding to a receptor, and that prevents an increase in arthritic score when administered to a mouse of the Tg197 transgenic mouse model of arthritis, wherein the single domain antibody polypeptide construct neutralizes human TNFα as measured in a standard L929 cell assay.

The invention further encompasses a composition comprising a single domain antibody polypeptide construct that antagonizes human TNFα's binding to a receptor, that prevents an increase in arthritic score when administered to a mouse of the Tg197 transgenic mouse model of arthritis, wherein the single domain antibody polypeptide construct inhibits the progression of the rheumatoid arthritis.

The invention further encompasses a composition comprising a single domain antibody polypeptide construct that antagonizes human TNFα's binding to a receptor, that prevents an increase in arthritic score when administered to a mouse of the Tg197 transgenic mouse model of arthritis, wherein the single domain antibody polypeptide construct binds human TNFα with a Kd of < 100 nM.

The invention further encompasses a composition comprising a single domain antibody polypeptide construct that antagonizes human TNFα's binding to a receptor, that prevents an increase in arthritic score when administered to a mouse of the Tg197 transgenic mouse model of arthritis, wherein the single domain antibody polypeptide construct neutralizes human TNFα as measured in a standard L929 cell assay, wherein the single domain antibody polypeptide construct inhibits the progression of the rheumatoid arthritis, wherein the single domain antibody polypeptide construct binds human TNFα with a Kd of < 100 nM.

In one embodiment the composition prevents an increase in arthritic score when administered to a mouse from a collagen induced arthritis (CIA) mouse model. Immunization of DBA/1 mice with murine type II collagen induces a chronic relapsing polyarthritis that provides a strong model for human autoimmune arthritis. The model is described, for example, by Courtenay et al., 1980, Nature 282 :666-668, Kato et al., 1996, Ann. Rheum. Dis. 55 :535-539 and Myers et al., 1997, Life Sci. 61:1861-1878, each of which is incorporated herein by reference.

In one embodiment the administration of the composition to the mouse comprises the following steps: a) administer weekly intraperitoneal injections of the composition to the CIA mouse, b) weigh the mouse of step a) weekly, and c) score the mouse weekly for macrophenotypic signs of arthritis according to the following system: 0 = no arthritis (normal appearance and flexion), 1 = mild arthritis (joint distortion), 2 = moderate arthritis (swelling, joint deformation), 3 = heavy arthritis (severely impaired movement).

In one embodiment the treating comprises inhibiting the progression of the rheumatoid arthritis.

In one embodiment the treating comprises preventing or delaying the onset of rheumatoid arthritis.

In one embodiment the administering results in a statistically significant change in one or more indicia of RA. The change is preferably by at least 10% or more.

In one embodiment the one or more indicia of RA comprise one or more of erythrocyte sedimentation rate (ESR), Ritchie particular index (described in Ritchie et al., 1968, Q. J. Med. 37: 393-406) and duration of morning stiffness, joint mobility, joint swelling, analysis by x ray imaging of one or more joints, and histopathological indications by analysis of fixed sections of one or more joints. Disease activity and change effected with treatment can also be evaluated using the disease activity score (DAS) and/or the chronic arthritis systemic index (CASI), see Carotti et al., 2002, Ann. Rheum. Dis. 61:877-882, and Salaffi et al., 2000, Rheumatology 39: 90-96.

In one embodiment the one or more indicia of RA comprises a decrease in the macrophenotypic signs of arthritis in a mouse from a collagen induced arthritis mouse model, wherein the composition is administered to the mouse, wherein the mouse is scored for the macrophenotypic signs of arthritis, and wherein the macrophenotypic signs of arthritis are scored according to the following system: 0 = no arthritis (normal appearance and flexion), 1 = mild arthritis (joint distortion), 2 = moderate arthritis (swelling, joint deformation), 3 = heavy arthritis (severely impaired movement).

In one embodiment the one or more indicia of RA comprises a decrease in the histopathological signs of arthritis in a mouse from a collagen induced arthritis mouse model, wherein the composition is administered to the mouse, wherein the mouse is scored for the histopathological signs of arthritis, and wherein the histopathological signs of arthritis are performed on a joint and scored using the following system: 0= no detectable pathology, 1= hyperplasia of the synovial membrane and presence of polymorphonuclear infiltrates, 2= pannus and fibrous tissue formation and focal subchondral bone erosion, 3= articular cartilage destruction and bone erosion, 4= extensive articular cartilage destruction and bone erosion.

In one embodiment the single domain antibody polypeptide construct is linked to a PEG molecule.

In one embodiment the PEG-linked single domain antibody polypeptide construct has a hydrodynamic size of at least 24 kDa, and wherein the total PEG size is from 20 to 60 kDa.

In one embodiment the PEG-linked single domain antibody polypeptide construct has a hydrodynamic size of at least 200 kDa and a total PEG size of from 20 to 60 kDa.

In one embodiment the PEGylated proteins of the invention may be linked, on average, to 1, 2, 3, 4, 5, 6, 7, 8, 9, 10, 12, 15, 17, 20, or more polyethylene glycol molecules.

### BRIEF DESCRIPTION OF THE FIGURES

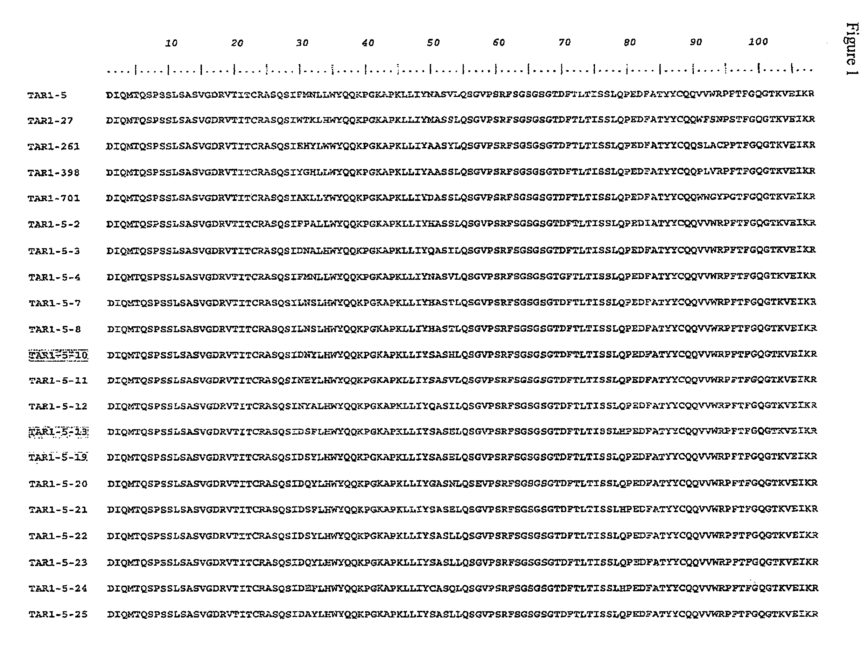

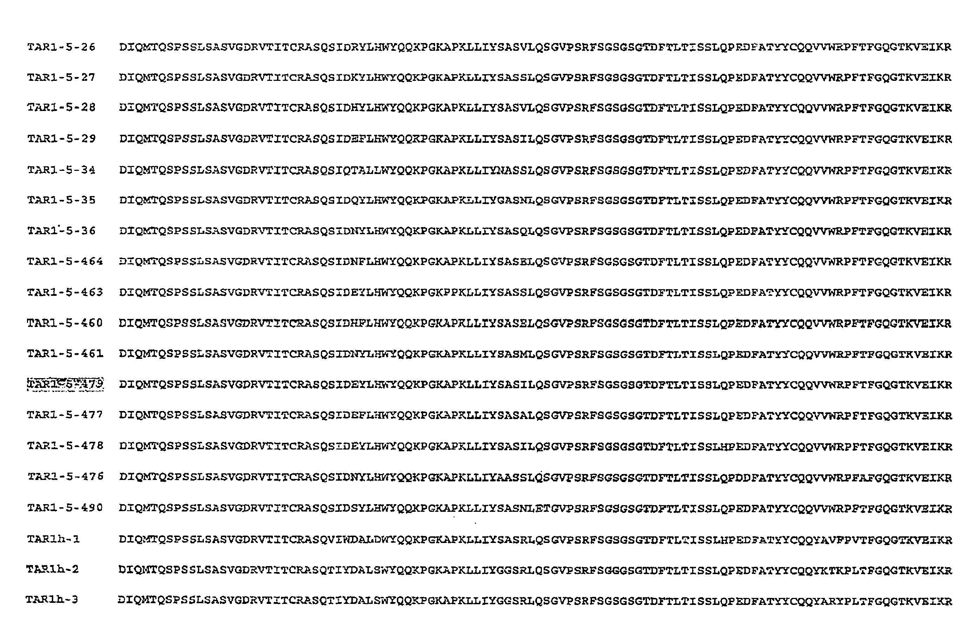

**Figure 1** Shows amino acid sequences for the TAR1 clones described herein (see, e.g., Example 1). TAR1-5, SEQ ID NO: 241; TAR1-27, SEQ ID NO: 242; TAR1-261, SEQ ID NO: 243; TAR1-398, SEQ ID NO: 244; TAR1-701, SEQ ID NO: 245; TAR1-5-2, SEQ ID NO: 246; TAR1-5-3, SEQ ID NO: 247; TAR1-5-4, SEQ ID NO: 248; TAR1-5-7, SEQ ID NO: 249; TAR1-5-8, SEQ ID NO: 250; TAR1-5-10, SEQ ID NO: 251; TAR1-5-11, SEQ ID NO: 252; TAR1-5-12, SEQ ID NO: 253; TAR1-5-13, SEQ ID NO: 254; TAR1-5-19, SEQ ID NO: 191; TAR1-5-20, SEQ ID NO: 255; TAR1-5-21, SEQ ID NO: 256; TAR1-5-22, SEQ ID NO: 257; TAR1-5-23, SEQ ID NO: 258; TAR1-5-24, SEQ ID NO: 259; TAR1-5-25, SEQ ID NO: 260; TAR1-5-26, SEQ ID NO: 261; TAR1-5-27, SEQ ID NO: 262; TAR1-5-28, SEQ ID NO: 263; TAR1-5-29, SEQ ID NO: 264; TAR1-5-34, SEQ ID NO: 265; TAR1-5-35, SEQ ID NO: 266; TAR1-5-36, SEQ ID NO: 267; TAR1-5-4G4, SEQ ID NO: 268; TAR1-5-463, SEQ ID NO: 269; TAR1-5-460, SEQ ID NO: 270; TAR1-5-461, SEQ ID NO: 271; TAR1-5-479, SEQ ID NO: 272; TAR1-5-477, SEQ ID NO: 273; TAR1-5-478, SEQ ID NO: 274; TAR1-5-476, SEQ ID NO: 275; TAR1-5-490, SEQ ID NO: 276; TAR1h-1, SEQ ID NO: 277; TAR1h-2, SEQ ID NO: 278; TAR1h-3, SEQ ID NO: 279.

### DETAILED DESCRIPTION

### Preparation of dAbs:

The invention relates to various constructs of ligands that bind TNF-α. The generation of such dAbs is discussed below and in the Examples.

In various aspects, the dAbs disclosed herein can be present in monomeric form, dimeric form, trimeric form, tetrameric form, or even in higher multimeric forms.

Single immunoglobulin variable domains or dAbs are prepared in a number of ways. In a preferred aspect, the dAbs arc human single immunoglobulin variable domains. For each of these approaches, well-known methods of preparing (e.g., amplifying, mutating, etc.) and manipulating nucleic acid sequences are applicable.

One means of preparing dAbs is to amplify and express the V_{H} or V_{L} region of a heavy chain or light chain gene for a cloned antibody known to bind the desired antigen. The boundaries of V_{H} and V_{L} domains are set out by Kabat et al. (1991, supra). The information regarding the boundaries of the V_{H} and V_{L} domains of heavy and light chain genes is used to design PCR primers that amplify the V domain from a cloned heavy or light chain coding sequence encoding an antibody known to bind a given antigen. The amplified V domain is inserted into a suitable expression vector, e.g., pHEN-1 (Hoogenboom et al., 1991, Nucleic Acids Res. 19: 4133-4137) and expressed, either alone or as a fusion with another polypeptide sequence. The expressed V_{H} or V_{L} domain is then screened for high affinity binding to the desired antigen in isolation from the remainder of the heavy or light chain polypeptide. For all aspects of the present invention, screening for binding is performed as known in the art or as described herein below.

A repertoire of V_{H} or V_{L} domains is screened by, for example, phage display, panning against the desired antigen. Methods for the construction of bacteriophage display libraries and lambda phage expression libraries are well known in the art, and taught, for example, by: McCafferty et al., 1990, Nature 348: 552; Kang et al., 1991, Proc. Natl. Acad. Sci. U.S.A., 88: 4363; Clackson et al., 1991, Nature 352: 624; Lowman et al., 1991, Biochemistry 30: 10832; Burton et al., 1991, Proc. Natl. Acad. Sci U.S.A. 88: 10134; Hoogenboom et al., 1991, Nucleic Acids Res. 19: 4133; Chang et al.,1991, J. Immunol. 147: 3610; Breitling et al., 1991, Gene 104: 147; Marks ct al., 1991, J. Mol. Biol. 222: 581; Barbas et al., 1992, Proc. Natl. Acad. Sci. U.S.A. 89: 4457; Hawkins and Winter (1992) J. Immunol., 22: 867; Marks et al. (1992) J. Biol. Chem., 267: 16007; and Lerner et al. (1992) Science, 258: 1313. scFv phage libraries are taught, for example, by Huston et al., 1988, Proc. Natl. Acad. Sci U.S.A. 85: 5879-5883; Chaudhary et al., 1990, Proc. Natl. Acad. Sci U.S.A. 87: 1066-1070; McCafferty et al., 1990, supra; Clackson et al.,1991, supra; Marks et al., 1991, supra; Chiswell et al., 1992, Trends Biotech. 10: 80; and Marks et al., 1992, supra. Various embodiments of scFv libraries displayed on bacteriophage coat proteins have been described. Refinements of phage display approaches are also known, for example as described in WO96/06213 and WO92/01047 (Medical Research Council et al.) and WO97/08320 (Morphosys, supra).

The repertoire of V_{H} or V_{L} domains can be a naturally-occurring repertoire of immunoglobulin sequences or a synthetic repertoire. A naturally-occurring repertoire is one prepared, for example, from immunoglobulin-expressing cells harvested from one or more individuals. Such repertoires can be "naïve," i.e., prepared, for example, from human fetal or newborn immunoglobulin-expressing cells, or rearranged, i.e., prepared from, for example, adult human B cells. Natural repertoires are described, for example, by Marks et al., 1991, J. Mol. Biol. 222: 581 and Vaughan et al., 1996, Nature Biotech. 14: 309. If desired, clones identified from a natural repertoire, or any repertoire, for that matter, that bind the target antigen are then subjected to mutagenesis and further screening in order to produce and select variants with improved binding characteristics.

Synthetic repertoires of single immunoglobulin variable domains are prepared by artificially introducing diversity into a cloned V domain. Synthetic repertoires are described, for example, by Hoogenboom & Winter, 1992, J. Mol. Biol. 227: 381; Barbas et al., 1992, Proc. Natl. Acad. Sci. U.S.A. 89: 4457; Nissim et al., 1994, EMBO J. 13: 692; Griffiths et al., 1994, EMBO J. 13: 3245; DeKriuf et al., 1995, J. Mol. Biol. 248: 97; and WO 99/20749.

The antigen binding domain of a conventional antibody comprises two separate regions: a heavy chain variable domain (V_{H}) and a light chain variable domain (V_{L}: which can be either V_{κ} or V_{λ}). The antigen binding site of such an antibody is formed by six polypeptide loops: three from the V_{H} domain (H1, H2 and H3) and three from the V_{L} domain (L1, L2 and L3). The boundaries of these loops are described, for example, in Kabat et al. (1991, supra). A diverse primary repertoire of V genes that encode the V_{H} and V_{L} domains is produced *in vivo* by the combinatorial rearrangement of gene segments. The V_{H} gene is produced by the recombination of three gene segments, V_{H}, D and J_{H}. In humans, there are approximately 51 functional V_{H} segments (Cook and Tomlinson (1995) Immunol Today 16: 237), 25 functional D segments (Corbett et al. (1997) J. Mol. Biol. 268: 69) and 6 functional J_{H} segments (Ravetch et al. (1981) Cell 27: 583), depending on the haplotype. The V_{H} segment encodes the region of the polypeptide chain which forms the first and second antigen binding loops of the V_{H} domain (H1 and H2), while the V_{H}, D and J_{H} segments combine to form the third antigen binding loop of the V_{H} domain (H3).

The V_{L} gene is produced by the recombination of only two gene segments, V_{L} and J_{L}. In humans, there are approximately 40 functional V_{κ} segments (Schäble and Zachau (1993) Biol. Chem. Hoppe-Seyler 374: 1001), 31 functional V_{λ} segments (Williams et al. (1996) J. Mol. Biol. 264: 220; Kawasaki et al. (1997) Genome Res. 7: 250), 5 functional J_{κ} segments (Hieter et al. (1982) J. Biol. Chem. 257: 1516) and 4 functional J_{λ} segments (Vasicek and Leder (1990) J. Exp. Med. 172: 609), depending on the haplotype. The V_{L} segment encodes the region of the polypeptide chain which forms the first and second antigen binding loops of the V_{L} domain (L1 and L2), while the V_{L} and J_{L} segments combine to form the third antigen binding loop of the V_{L} domain (L3). Antibodies selected from this primary repertoire are believed to be sufficiently diverse to bind almost all antigens with at least moderate affinity. High affinity antibodies are produced *in vivo* by "affinity maturation" of the rearranged genes, in which point mutations are generated and selected by the immune system on the basis of improved binding.

Analysis of the structures and sequences of antibodies has shown that five of the six antigen binding loops (H1, H2, L1, L2, L3) possess a limited number of main-chain conformations or canonical structures (Chothia and Lesk (1987) J. Mol. Biol. 196: 901; Chothia et al. (1989) Nature 342: 877). The main-chain conformations are determined by (i) the length of the antigen binding loop, and (ii) particular residues, or types of residue, at certain key position in the antigen binding loop and the antibody framework. Analysis of the loop lengths and key residues has enabled us to the predict the main-chain conformations of H1, H2, L1, L2 and L3 encoded by the majority of human antibody sequences (Chothia et al. (1992) J. Mol. Biol. 227: 799; Tomlinson et al. (1995) EMBO J. 14: 4628; Williams et al. (1996) J. Mol. Biol. 264: 220). Although the H3 region is much more diverse in terms of sequence, length and structure (due to the use of D segments), it also forms a limited number of main-chain conformations for short loop lengths which depend on the length and the presence of particular residues, or types of residue, at key positions in the loop and the antibody framework (Martin et al. (1996) J. Mol. Biol. 263: 800; Shirai et al. (1996) FEBS Letters 399: 1.

While, according to one embodiment of the invention, diversity can be added to synthetic repertoires at any site in the CDRs of the various antigen-binding loops, this approach results in a greater proportion of V domains that do not properly fold and therefore contribute to a lower proportion of molecules with the potential to bind antigen. An understanding of the residues contributing to the main chain conformation of the antigen-binding loops permits the identification of specific residues to diversify in a synthetic repertoire of V_{H} or V_{L} domains. That is, diversity is best introduced in residues that are not essential to maintaining the main chain conformation. As an example, for the diversification of loop L2, the conventional approach would be to diversify all the residues in the corresponding CDR (CDR2) as defined by Kabat et al. (1991, supra), some seven residues. However, for L2, it is known that positions 50 and 53 are diverse in naturally occurring antibodies and are observed to make contact with the antigen. The preferred approach would be to diversify only those two residues in this loop. This represents a significant improvement in terms of the functional diversity required to create a range of antigen binding specificities.

In one aspect, synthetic variable domain repertoires are prepared in V_{H} or Vκ backgrounds, based on artificially diversified germline V_{H} or Vκ sequences. For example, the V_{H} domain repertoire is based on cloned germ line V_{H} gene segments V3-23/DP47 (Tomlinson et al., 1992, J. Mol. Biol. 227: 7768) and JH4b (see Figures 1 and 2). The V_{κ} domain repertoire is based, for example, on germline V_{κ} gene segments O2/O12/DPK9 (Cox et al., 1994, Eur. J. Immunol. 24: 827) and J_{κ}1 (see Figure 3). Diversity is introduced into these or other gene segments by, for example, PCR mutagenesis. Diversity can be randomly introduced, for example, by error prone PCR (Hawkins, et al., 1992, J. Mol. Biol. 226: 889) or chemical mutagenesis. As discussed above, however it is preferred that the introduction of diversity is targeted to particular residues. It is further preferred that the desired residues are targeted by introduction of the codon NNK using mutagenic primers (using the IUPAC nomenclature, where N = G, A, T or C, and K = G or T), which encodes all amino acids and the TAG stop codon. Other codons which achieve similar ends are also of use, including the NNN codon (which leads to the production of the additional stop codons TGA and TAA), DVT codon ((A/G/T) (A/G/C)T), DVC codon ((A/G/T)(A/G/C)C), and DVY codon ((A/G/T)(A/G/C)(C/T). The DVT codon encodes 22% serine and 11% tyrosine, asgpargine, glycine, alanine, aspartate, threonine and cysteine, which most closely mimics the distribution of amino acid residues for the antigen binding sites of natural human antibodies. Repertoires are made using PCR primers having the selected degenerate codon or codons at each site to be diversified. PCR mutagenesis is well known in the art.

### Screening dAbs for Antigen Binding:

Following expression of a repertoire of dAbs on the surface of phage, selection is performed by contacting the phage repertoire with immobilized target antigen, washing to remove unbound phage, and propagation of the bound phage, the whole process frequently referred to as "panning." Alternatively, phage are pre-selected for the expression of properly folded member variants by panning against an immobilized generic ligand (e.g., protein A or protein L) that is only bound by folded members. This has the advantage of reducing the proportion of non-functional members, thereby increasing the proportion of members likely to bind a target antigen. Pre-selection with generic ligands is taught in WO 99/20749. The screening of phage antibody libraries is generally described, for example, by Harrison et al., 1996, Meth. Enzymol. 267: 83-109.

Screening is commonly performed using purified antigen immobilized on a solid support, for example, plastic tubes or wells, or on a chromatography matrix, for example Sepharose^{™} (Pharmacia). Screening or selection can also be performed on complex antigens, such as the surface of cells (Marks et al., 1993, BioTechnology 11: 1145; de Kruif et al., 1995, Proc. Natl. Acad. Sci. U.S.A. 92: 3938). Another alternative involves selection by binding biotinylated antigen in solution, followed by capture on streptavidin-coated beads.

In a preferred aspect, panning is performed by immobilizing antigen (generic or specific) on tubes or wells in a plate, e.g., Nunc MAXISORP^{™} immunotube 8 well strips. Wells are coated with 150 µl of antigen (100 µg/ml in PBS) and incubated overnight. The wells are then washed 3 times with PBS and blocked with 400 µl PBS-2% skim milk (2%MPBS) at 37°C for 2 hr. The wells are rinsed 3 times with PBS and phage are added in 2%MPBS. The mixture is incubated at room temperature for 90 minutes and the liquid, containing unbound phage, is removed. Wells are rinsed 10 times with PBS-0.1 % tween 20, and then 10 times with PBS to remove detergent. Bound phage are eluted by adding 200 µl of freshly prepared 100 mM triethylamine, mixing well and incubating for 10 min at room temperature. Eluted phage are transferred to a tube containing 100 µl of I M Tris-HCl, pH 7.4 and vortexed to neutralize the triethylamine. Exponentially-growing E. coli host cells (e.g., TG1) are infected with, for example, 150 ml of the eluted phage by incubating for 30 min at 37°C. Infected cells are spun down, resuspended in fresh medium and plated in top agarose. Phage plaques are eluted or picked into fresh cultures of host cells to propagate for analysis or for further rounds of selection. One or more rounds of plaque purification are performed if necessary to ensure pure populations of selected phage. Other screening approaches are described by Harrison et al., 1996, *supra.*

Following identification of phage expressing a single immunoglobulin variable domain that binds a desired target, if a phagemid vector such as pHEN1 has been used, the variable domain fusion protein are easily produced in soluble form by infecting non-suppressor strains of bacteria, e.g., HB2151 that permit the secretion of soluble gene III fusion protein. Alternatively, the V domain sequence can be sub-cloned into an appropriate expression vector to produce soluble protein according to methods known in the art.

### Purification and Concentration of dAbs:

dAb polypeptides secreted into the periplasmic space or into the medium of bacteria are harvested and purified according to known methods (Harrison et al., 1996, supra). Skerra & Pluckthun (1988, Science 240: 1038) and Breitling et al. (1991, Gene 104: 147) describe the harvest of antibody polypeptides from the periplasm, and Better et al. (1988, Science 240: 1041) describes harvest from the culture supernatant. Purification can also be achieved by binding to generic ligands, such as protein A or Protein L. Alternatively, the variable domains can be expressed with a peptide tag, e.g., the Myc, HA or 6X-His tags, which facilitates purification by affinity chromatography.

In particular the invention provides an anti-TNF-α dAb monomer (or dual specific ligand comprising such a dAb), homodimer, heterodimer or homotrimer ligand, wherein each dAb binds TNF-α. The ligand binds to TNF-α with a K_{d} of 300nM to 5pM (ie, 3 x 10⁻⁷ to 5 x 10⁻¹²M), preferably 50nM to 20pM, more preferably 5nM to 200pM and most preferably 1nM to 100pM; expressed in an alternative manner, the K_{d} is 1 x 10⁻⁷ M or less, preferably 1 x 10⁻⁸ M or less, more preferably 1 x 10⁻⁹ M or less, advantageously 1 x 10⁻¹⁰ M or less and most preferably 1 x 10⁻¹¹ M or less; and/or a K_{off} rate constant of 5 x 10⁻¹ to 1 x 10⁻⁷ S⁻¹, preferably I x 10⁻² to 1 x 10⁻⁶ S⁻¹, more preferably 5 x 10⁻³ to I x 10⁻⁵ S⁻¹, for example 5 x 10⁻¹S⁻¹ or less, preferably 1 x 10⁻² S⁻¹ or less, more preferably 1 x 10⁻³ S⁻¹ or less, advantageously 1 x 10⁻⁴ S⁻¹ or less, further advantageously 1 x 10⁻⁵ S⁻¹ or less, and most preferably 1 x 10⁻⁶ S⁻¹ or less, as determined by surface plasmon resonance.

Preferably, the ligand neutralises TNF-α in a standard L929 assay with an ND50 of 500nM to 50pM, preferably or 100nM to 50pM, advantageously 10nM to 100pM, more preferably 1nM to 100pM; for example 50nM or less, preferably 5nM or less, advantageously 500pM or less, more preferably 200pM or less and most preferably 100pM or less.

Preferably, the ligand inhibits binding of TNF-α to TNF-α Receptor 1 (p55 receptor) with an IC50 of 500nM to 50pM, preferably 100nM to 50pM, more preferably 5 10nM to 100pM, advantageously 1nm to 104pM; for example 50nM or less, preferably 5nM or less, more preferably 500pM or less, advantageously 200pM or less, and most preferably 100pM or less. Preferably, the TNF-α is Human TNF-α.

Preferably, the dAb monomer or ligand neutralises TNF-α in a standard assay (eg, the L929 or HeLa assays described herein) with an ND50 of 500nM to 50pM, preferably 100nM to 50pM, more preferably 10nM to 100pM, advantageously 1nM to 100pM; for example 50nM or less, preferably 5nM or less, more preferably 500pM or less, advantageously 200pM or less, and most preferably 100pM or less.

The invention moreover provides dimers, trimers and polymers of the aforementioned dAb monomers, in accordance with the foregoing aspect of the present invention.

Ligands according to the invention, including dAb monomers, dimers and trimers, can be linked to an antibody Fc region, comprising one or both of CH2 and CH3 domains, and optionally a hinge region. For example, vectors encoding ligands linked as a single nucleotide sequence to an Fc region may be used to prepare such polypeptides.

### Multimeric Forms of Antibody Single Variable Domains:

In one aspect, an antibody polypeptide construct (e.g., a dAb) as described herein is multimerized, as for example, homodimers, homotrimers, homotetramers, or higher order homomultimers (e.g., homo-pentamer and up to octomers). Multimerization can increase the strength of antigen binding through the avidity effect, wherein the strength of binding is related to the sum of the binding affinities of the multiple binding sites.

### PEGylation of Antibody Polypeptides

The present invention provides PEGylated antibody polypeptide (e.g., dAb) monomers and multimers which provide increased half-life and resistance to degredation without a loss in activity (e.g., binding affinity) relative to non-PFGylated antibody polypeptides.

Antibody polypeptide molecules as described herein can be coupled, using methods known in the art, to polymer molecules (preferably PEG) useful for achieving the increased half-life and degradation resistance properties. Polymer moieties which can be utilized in the invention can be synthetic or naturally occurring and include, but are not limited to straight or branched chain polyalkylene, polyalkenylene or polyoxyalkylene polymers, or a branched or unbranched polysaccharide such as a homo- or heteropolysaccharide. Preferred examples of synthetic polymers which can be used in the invention include straight or branched chain poly(ethylene glycol) (PEG), poly(propylene glycol), or poly(vinyl alcohol) and derivatives or substituted forms thereof. Particularly preferred substituted polymers for linkage to antibody polypeptides as described herein include substituted PEG, including methoxy(polyethylene glycol). Naturally occurring polymer moieties which can be used in addition to or in place of PEG include lactose, amylose, dextran, or glycogen, as well as derivatives thereof which would be recognized by one of skill in the art. Derivatized forms of polymer molecules include, for example, derivatives which have additional moieties or reactive groups present therein to permit interaction with amino acid residues of the antibody polypeptides described herein. Such derivatives include N-hydroxylsuccinimide (NHS) active esters, succinimidyl propionate polymers, and sulfhydryl-selective reactive agents such as maleimide, vinyl sulfone, and thiol. Particularly preferred derivatized polymers include, but are not limited to PEG polymers having the formulae: PEG-O-CH₂CH₂CH₂-CO₂-NHS; PEG-O-CH₂-NHS; PEG-O-CH₂CH₂-CO₂-NHS; PEG-S-CH₂CH₂-CO-NHS; PEG-O₂CNH-CH(R)-CO₂-NHS; PEG-NHCO-CH₂CH₂-CO-NHS; and PEG-O-CH₂-CO₂-NHS; where R is (CH₂)₄)NHCO₂(mPEG). PEG polymers can be linear molecules, or can be branched wherein multiple PEG moieties arc present in a single polymer.

The ligands or binding proteins thereof, for example dAb monomers, of the present invention will typically find use in preventing, suppressing or treating inflammatory states, allergic hypersensitivity, cancer, bacterial or viral infection, and autoimmune disorders (which include, but are not limited to, Type 1 diabetes, asthma, multiple sclerosis, rheumatoid arthritis, systemic lupus erythematosus, Crohn's disease and myasthenia gravis).

In addition to rheumatoid arthritis, anti-TNP-alpha polypeptides as described herein are applicable to the treatment of autoimmune diseases, such as (parentheticals indicate affected organ), but not limited to: Addison's disease (adrenal); autoimmune diseases of the ear (ear); autoimmune diseases of the eye (eye); autoimmune hepatitis (liver); autoimmune parotitis (parotid glands); Crohn's disease and inflammatory bowel disease (intestine); Diabetes Type I (pancreas); epididymitis (epididymis), glomerulonephritis (kidneys); Graves' disease (thyroid); Guillain-Barre syndrome (nerve cells); Hashimoto's disease (thyroid); hemolytic anemia (red blood cells); systemic lupus erythematosus (multiple tissues); male infertility (sperm); multiple sclerosis (nerve cells); myasthenia gravis (neuromuscular junction); pemphigus (primarily skin); psoriasis (skin); rheumatic fever (heart and joints); sarcoidosis (multiple tissues and organs); scleroderma (skin and connective tissues); Sjogren's syndrome (exocrine glands, and other tissues); spondyloarthropathies (axial skeleton, and other tissues); thyroiditis (thyroid); ulcerative colitis (intestine); and vasculitis (blood vessels).

In addition to rheumatoid arthritis and other chronic inflammatory disorders (e.g., Crohn's disease, psoriasis, etc.), anti-VEGF polypeptides as described herein can be used to treat diabetes, acute myeloid leukemia, leukemia and ophthalmic disorders, including macular degeneration and diabetic retinopathy.

In the instant application, the term 'prevention" involves administration of the protective composition prior to the induction of the disease. "Suppression" refers to administration of the composition after an inductive event, but prior to the clinical appearance of the disease.

Animal model systems which can be used to screen the effectiveness of the antibodies or binding proteins thereof in protecting against or treating the disease are available. Methods for the testing of systemic lupus erythematosus (SLE) in susceptible mice are known in the art (Knight et al. (1978) J. Exp. Med., 147: 1653; Reinersten et al. (1978) New Eng. J. Med., 299: 515). Myasthenia Gravis (MG) is tested in SJL/J female mice by inducing the disease with soluble AchR protein from another species (Lindstrom et al. (1988) Adv. Immurzol., 42: 233). Arthritis is induced in a susceptible strain of mice by 30 injection of Type II collagen (Stuart et al. (1984) Ann. Rev. Immunol., 42: 233). A model by which adjuvant arthritis is induced in susceptible rats by injection of mycobacterial heat shock protein has been described (Van Eden et al. (1988) Nature, 331: 171). Thyroiditis is induced in mice by administration of thyroglobulin as described (Maron et al. (1980) J. Exp. Med., 152: 1115). Insulin dependent diabetes mellitus (IDDM) occurs naturally or can be induced in certain strains of mice such as those described by Kanasawa et al. (1984) Diabetologia, 27: 113. EAE in mouse and rat serves as a model for MS in human. In this model, the demyelinating disease is induced by administration of myelin basic protein (see Paterson (1986) Textbook of Immuopathology, Mischer et al., eds., Grune and Stratton, New York, pp. 179-213; McFarlin et al. (1973) Science, 179: 478: and Satoh et al. (1987) J. Immunol., 138: 179).

Generally, the present ligands will be utilised in purified form together with pharmacologically appropriate carriers. Typically, these carriers include aqueous or alcoholic/aqueous solutions, emulsions or suspensions, any including saline and/or buffered media. Parenteral vehicles include sodium chloride solution, Ringer's dextrose, dextrose and sodium chloride and lactated Ringer's. Suitable physiologically-acceptable adjuvants, if necessary to keep a polypeptide complex in suspension, may be chosen from thickeners such as carboxymethylcellulose, polyvinylpyrrolidone, gelatin and alginates.

Intravenous vehicles include fluid and nutrient replenishers and electrolyte replenishers, such as those based on Ringer's dextrose. Preservatives and other additives, such as antimicrobials, antioxidants, chelating agents and inert gases, may also be present (Mack (1982) Remington's Pharmaceutical Sciences, 16th Edition).

The ligands of the present invention may be used as separately administered compositions or in conjunction with other agents. These can include various immunotherapeutic drugs, such as cylcosporine, methotrexate, adriamycin or cisplatinum, and immunotoxins. Pharmaceutical compositions can include "cocktails" of various cytotoxic or other agents in conjunction with the ligands of the present invention, or even combinations of lignds according to the present invention having different specificities, such as ligands selected using different target antigens or epitopes, whether or not they are pooled prior to administration.

The route of administration of pharmaceutical compositions according to the invention may be any of those commonly known to those of ordinary skill in the art. For therapy, including without limitation immunotherapy, the selected ligands thereof of the invention can be administered to any patient in accordance with standard techniques. The administration can be by any appropriate mode, including parenterally, intravenously, intramuscularly, intraperitoneally, transdermally, via the pulmonary route, or also, appropriately, by direct infusion with a catheter. The dosage and frequency of administration will depend on the age, sex and condition of the patient, concurrent administration of other drugs, counterindications and other parameters to be taken into account by the clinician.

As will be appreciated by the skilled artisan, the route and/or mode of administration will vary depending upon the desired results.

The compositions containing the present ligands or a cocktail thereof can be administered for prophylactic andlor therapeutic treatments. In certain therapeutic applications, an adequate amount to accomplish at least partial inhibition, suppression, modulation, killing, or some other measurable parameter, of a population of selected cells is defined as a "therapeutically-effective dose". Amounts needed to achieve this dosage will depend upon the severity of the disease and the general state of the patient's own immune system, but generally range from 0.005 to 5.0 mg of ligand, e.g. antibody, receptor (e.g. a T-cell receptor) or binding protein thereof per kilogram of body weight, with doses of 0.05 to 2.0 mg/kg/dose being more commonly used. For prophylactic applications, compositions containing the present ligands or cocktails thereof may also be administered in similar or slightly lower dosages.

Treatment performed using the compositions described herein is considered "effective" if one or more symptoms is reduced (e.g., by at least 10% or at least one point on a clinical assessment scale), relative to such symptoms present before treatment, or relative to such symptoms in an individual (human or model animal) not treated with such composition. Symptoms will obviously vary depending upon the disease or disorder targeted, but can be measured by an ordinarily skilled clinician or technician. Such symptoms can be measured, for example, by monitoring the level of one or more biochemical indicators of the disease or disorder (e.g., levels of an enzyme or metabolite correlated with the disease, affected cell numbers, etc.), by monitoring physical manifestations (e.g., inflammation, tumor size, etc.), or by an accepted clinical assessment scale, for example, the Expanded Disability Status Scale (for multiple sclerosis), the Irvine Inflammatory Bowel Disease Questionnaire (32 point assessment evaluates quality of life with respect to bowel function, systemic symptoms, social function and emotional status - score ranges from 32 to 224, with higher scores indicating a better quality of life), the Quality of Life Rheumatoid Arthritis Scale, or other accepted clinical assessment scale as known in the field. A sustained (e.g., one day or more, preferably longer) reduction in disease or disorder symptoms by at least 10% or by one or more points on a given clinical scale is indicative of "effective" treatment. Similarly, prophylaxis performed using a composition as described herein is "effective" if the onset or severity of one or more symptoms is delayed, reduced or abolished relative to such symptoms in a similar individual (human or animal model) not treated with the composition.

A composition containing a ligand or cocktail thereof according to the present invention may be utilised in prophylactic and therapeutic settings to aid in the alteration, inactivation, killing or removal of a select target cell population in a mammal. In addition, the selected repertoires of polypeptides described herein may be used exhacorporeally or in vitro selectively to kill, deplete or otherwise effectively remove a target cell population from a heterogeneous collection of cells. Blood from a mammal may be combined extracorporeally with the ligands, e.g. antibodies, cell-surface receptors or binding proteins thereof whereby the undesired cells are killed or otherwise removed from the blood for return to the mammal in accordance with standard techniques.

In a preferred embodiment, ligands as described herein can be used to treat rheumatoid arthritis.

In one aspect, the invention provides methods of treating rheumatoid arthritis, comprising the use of one or more single domain antibody polypeptide constructs, wherein one or more of the constructs antagonizes human TNFα's binding to a receptor. The present invention encompasses compositions comprising one or more single domain antibody polypeptide constructs that antagonize human TNFα's binding to a receptor.

In one embodiment the invention provides methods of treatment of rheumatoid arthritis comprising administering a composition comprising one or more single domain antibody polypeptide constructs, wherein one or more of the constructs antagonizes human TNFα's binding to a receptor, and/or prevents all increase in arthritic score when administered to a mouse of the Tg197 transgenic mouse model of arthritis, and/or neutralizes TNF-α in the L929 cytotoxicity assay. In particular, methods of treatment of arthritis comprise the administration of a composition comprising one or more single domain antibody polypeptide constructs, wherein one or more of the constructs antagonizes human TNFα's binding to a receptor, and wherein the administration of the composition to a Tg197 transgenic mouse prevents an increase in arthritic score.

### a) Receptor Binding Assays

Ligands for the treatment of rheumatoid arthritis can interfere with the binding of TNF-α to a TNF-α receptor. The receptor can be an isolated (usually membrane-bound) receptor, or it can be a receptor present on a cell, either in vitro or in vivo.

As used herein, the term "antagonizes binding" of the receptor refers to the ability or effect of a given antibody polypeptide construct to interfere with the binding of TNF-α (or VEGF or other factor) to a cognate receptor. Antagonism is measured using one or more of the in vitro, cell-based or in vivo assays as described herein. Thus, the receptor can be isolated, membrane bound, or present on the cell surface. A construct interferes with or antagonizes binding to a cognate receptor (e.g., TNFR1, TNFR2, VEGFR1, VEGFR2) if there is a statistically significant decrease in binding detected in the presence of the construct relative to the absence of the construct. Alternatively, a construct interferes with binding if there is at least a 10% decrease in measured binding in the presence of the construct, relative to its absence.

### b) L929 cytotoxicity assay

Ligands for the treatment of rheumatoid arthritis can interfere with the cytotoxic effects of TNF-α in the L929 cytotoxicity assay. This assay, based on the assay described by Evans et al., 2000, Molecular Biotechnology 15: 243-248. Anti-TNF-α ligands useful for the treatment of rheumatoid arthritis can neutralize the activity of TNF-α in this cell assay.

As used herein, the term "neutralizing," when used in reference to an antibody or dAb polypeptide as described herein, means that the polypeptide interferes with a measurable activity or function of the target antigen. A polypeptide is a "neutralizing" polypeptide if it reduces a measurable activity or function of the target antigen by at least 50%, and preferably at least 60%, 70%, 80%, 90%, 95% or more, up to and including 100% inhibition (i.e., no detectable effect or function of the target antigen). Thus, where the target is TNF-α, neutralizing activity can be assessed using the standard L929 cell killing assay described herein or by measuring the ability of an anti-TNF-α polypeptide construct to inhibit TNF-α-induced expression of ELAM-1 on HUVEC, which measures TNF-α-induced cellular activation.

### 4. Treatment of Crohn's Disease

Anti-TNF-α polypeptides as described herein can be used to treat Crohn's disease in humans. In one embodiment the invention provides methods of treatment of Crohn's disease or other inflammatory bowel disease (IBD) in which TNF-α is involved. The methods comprise administering a composition comprising one or more single domain antibody polypeptide constructs, wherein one or more of the constructs antagonizes human TNFα's binding to a receptor, and/or prevents an increase in acute or chronic inflammatory bowel score when administered to a mouse of the Tnf^{ΔARE} transgenic mouse model of IBD, and/or neutralizes TNF-α in the L929 cytotoxicity assay. In particular, methods of treatment of Crohn's or other inflammatory bowel disorders comprise the administration of a composition comprising one or more single domain antibody polypeptide constructs, wherein one or more of the constructs antagonizes human TNFα's binding to a receptor, and wherein the administration of the composition to a Tnf^{ΔARE} transgenic mouse prevents an increase or effects a decrease in acute or chronic inflammatory bowel score.

### EXAMPLES

### Example 1: Summary of Nucleic Acid and Polypeptide Sequences for anti-TNF-α dAbs.

Throughout the course of studies regarding the anti-TNF-α dAbs described herein, a number of different dAbs have been identified that bind human and/or mouse TNF-α. Sequences and further information are provided herein below.

### dAb clones that bind human TNF-α

The following is a listing of the nucleotide sequences of dAbs identified for binding human TNF-α. Corresponding amino acid sequences are provided in **Figure 1****.**

### TAR1-5 (SEQ ID NO: 101)

GACATCCAGATGACCCAGTCTCCATCCTCCCTGTCTGCATCTGTAGGAGA
CCGTGTCACCATCACTTGCCGGGCAAGTCAGAGCATTTTTATGAATTTAT
TGTGGTACCAGCAGAAACCAGGGAAAGCCCCTAAGCTCCTGATCTATAAT
GCATCCGTGTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGC

### TAR1-27 (SEQ ID NO: 102)

GACATCCAGATGACCCAGTCTCCATCCTCCCTGTCTGCATCTGTAGGAGA
CCGTGTCACCATCACTTGCCGGGCAAGTCAGAGCATTTGGACGAAGTTAC
ATTGGTACCAGCAGAAACCAGGGAAAGCCCCTAAGCTCCTGATCTATATG
GCATCCAGTTTGCAAAGTGGGGTCCCATCACGTTTCAGTGGCAGTGGATC
TGGCACAGATTTCACTCTCACCATCAGCAGTCTGCAACCTGAAGATTTTG
CTACGTACTACTGTCAACAGTGGTTTAGTAATCCTAGTACGTTCGGCCAA
GGGACCAAGGTGGAAATCAAACG

### TAR1-261 (SEQ ID NO: 103)

GACATCCAGATGACCCAGTCTCCATCCTCCCTGTCTGCATCTGTAGGAGA
CCGTGTCACCATCACTTGCCGGGCAAGTCAGAGCATTGAGCATTATTTAT
GGTGGTACCAGCAGAAACCAGGGAAAGCCCCTAAGCTCCTGATCTATGCT
GCATCCTATTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGAGTTTGGCGTGTCCTCCTACGTTCGGCCAA
GGGACCAAGGTGGAAATCAAACGG

### TAR1-398 (SEQ ID NO: 104)

GACATCCAGATGACCCAGTCTCCATCCTCCCTGTCTGCATCTGTAGGAGA
CCGTGTCACCATCACTTGCCGGGCAAGTCAGAGCATTTATGGTCATTTAT
TGTGGTACCAGCAGAAACCAGGGAAAGCCCCTAAGCTCCTGATCTATGCT
GCATCCAGTTTGCAAAGTGGGGTCCCATCACGTTTCAGTGGCAGTGGATC
TGGGACAGATTTCACTCTCACCATCAGCAGTCTGCAACCTGAAGATTTTG

CTACGTACTACTGTCAACAGCCTTTGGTGCGGCCTTTTACGTTCGGCCAA
GGGACCAAGGTGGAAATCAAACGG

### TAR1-701 (SEQ ID NO: 105)

GACATCCAGATGACCCAGTCTCCATCCTCCCTGTCTGCATCTGTAGGAGA
CCGTGTCACCATCACTTGCCGGGCAAGTCAGAGCATTGCTAAGTTGTTAT
ATTGGTACCAGCAGAAACCAGGGAAAGCCCCTAAGCTCCTGATCTATGAT
GCATCCTCTTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGTGGTGGGGGTATCCTGGTACGTTCGGCCAA
GGGACCAAGGTGGAAATCAAACGG

### TAR1-5-2 (SEQ ID NO: 106)

GACATCCAGATGACCCAGTCTCCATCCTCCCTGTCTGCATCTGTAGGAGA
CCGTGTCACCATCACTTGCCGGGCAAGTCAGAGCATTTTTCCTGCTTTAC
TTTGGTACCAGCAGAAACCAGGGAAAGCCCCTAAGCTCCTGATCTATCAT
GCATCCAGTTTGCAAAGTGGGGTCCCATCACGTTTCAGTGGCAGTGGATC
TGGGACAGATTTCACTCTCACCATCAGCAGTCTGCAACCTGAAGATATTG
CTACGTACTACTGTCAACAGGTTGTGTGGCGTCCTTTTACGTTCGGCCAA
GGGACCAAGGTGGAAATCAAACGG

### TAR1-5-3 (SEQ ID NO: 107)

GACATCCAGATGACCCAGTCTCCATCCTCCCTGTCTGCATCTGTAGGAGA
CCGTGTCACCATCACTTGCCGGGCAAGTCAGAGCATTGATAATGCGTTAC
ATTGGTACCAGCAGAAACCAGGGAAAGCCCCTAAGCTCCTGATCTATCAG
GCATCCATTTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

### TAR1-3-4 (SEQ ID NO: 108)

GACATCCAGATGACCCAGTCTCCATCCTCCCTGTCTGCATCTGTAGGAGA
CCGTGTCACCATCACTTGCCGGGCAAGTCAGAGCATTTTTATGAATTTAT
TGTGGTACCAGCAGAAACCAGGGAAAGCCCCTAAGCTCCTGATCTATAAT
GCATCCGTGTTGCAAAGTGGGGTCCCATCACGTTTCAGTGGCAGTGGATC
TGGGACAGGTTTCACTCTCACCATCAGCAGTCTGCAACCTGAAGATTTTG
CTACGTACTACTGTCAACAGGTTGTGTGGCGTCCTTTTACGTTCGGCCAA

GGGACCAAGGTGGAAATCAAACGG

### TAR1-5-7 (SEQ ID NO: 109)

GACATCCAGATGACCCAGTCTCCATCCTCCCTGTCTGCATCTGTAGGAGA
CCGTGTCACCATCACTTGCCGGGCAAGTCAGAGCATTTTGAATTCTTTAC
ATTGGTACCAGCAGAAACCAGGGAAAGCCCCTAAGCTCCTGATCTATCAT
GCATCCACTTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

### TAR1-5-8 (SEQ ID NO: 110)

GACATCCAGATGACCCAGTCTCCATCCTCCCTGTCTGCATCTGTAGGAGA
CCGTGTCACCATCACTTGCCGGGCAAGTCAGAGCATTTTGAATTCTTTAC
ATTGGTACCAGCAGAAACCAGGGAAAGCCCCTAAGCTCCTGATCTATCAT
GCATCCACTTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

### TAR1-5-10 (SEQ ID NO: 111)

GACATCCAGATGACCCAGTCTCCATCCTCCCTGTCTGCATCTGTAGGAGA
CCGTGTCACCATCACTTGCCGGGCAAGTCAGAGCATTGATAATTATTTAC
ATTGGTACCAGCAGAAACCAGGGAAAGCCCCTAAGCTCCTGATCTATTCT
GCATCCCATTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

### TAR1-5-11 (SEQ ID NO: 112)

GACATCCAGATGACCCAGTCTCCATCCTCCCTGTCTGCATCTGTAGGAGA
CCGTGTCACCATCACTTGCCGGGCAAGTCAGAGCATTAATGAGTATTTAC
ATTGGTACCAGCAGAAACCAGGGAAAGCCCCTAAGCTCCTGATCTATTCT
GCATCCGTGTTGCAAAGTGGGGTCCCATCACGTTTCAGTGGCAGTGGATC
TGCGACACATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

### TAR1-5-12 (SEQ ID NO: 113)

GACATCCAGATGACCCAGTCTCCATCCTCCCTGTCTGCATCTCTAGGAGA
CCGTGTCACCATCACTTGCCGGGCAAGTCAGAGCATTAATTATGCTTTAC
ATTGGTACCAGCAGAAACCAGGGAAAGCCCCTAAGCTCCTGATCTATCAG
GCATCCATTTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

### TAR1-5-13 (SEQ ID NO: 114)

GACATCCAGATGACCCAGTCTCCATCCTCCCTGTCTGCATCTGTAGGAGA
CCGTGTCACCATCACTTGCCGGGCAAGTCAGAGCATTGATAGTTTTTTAC
ATTGGTACCAGCAGAAACCAGGGAAAGCCCCTAAGCTCCTGATCTATAGT
GCATCCGAGTTGCAAAGTGGGGTCCCATCACGTTTCAGTGGCAGTGGATC
TGCGACAGATTTCACTCTCACCATCAGCAGTCTGCATCCTGAAGATTTTG
CTACGTACTACTGTCAACAGGTTGTGTGGCGTCCTTTTACGTTCGGCCAA
GGGACCAAGGTGGAAATCAAACGG

### TAR1-5-19 (SEQ ID NO: 15)

GACATCCAGATGACCCAGTCTCCATCCTCTCTGTCTGCATCTGTAGGAGA
CCGTGTCACCATCACTTGCCGGGCAAGTCAGAGCATTGATAGTTATTTAC
ATTGGTACCAGCAGAAACCAGGGAAAGCCCCTAAGCTCCTGATCTATAGT
GCATCCGAGTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

### TAR1-5-20 (SEQ ID NO: 116)

GACATCCAGATGACCCAGTCTCCATCCTCCCTGTCTGCATCTGTAGGAGA
CCG[?]GTCACCATCACTTGCCGGGCAAGTCAGAGCATTGATCAGTATTTAC
ATTGGTACCAGCAGAAACCAGGGAAAGCCCCTAAGCTCCTGATCTATGGT
GCATCCAATTTGCAAAGTGAGGTCCCATCACGTTTCAGTGGCAGTGCATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

### TAR1-5-21 (SEQ ID NO: 117)

GACATCCAGATGACCCAGTCTCCATCCTCCCTGTCTGCATCTGTAGGAGA
CCGTGTCACCATCACTTGCCGGGCAAGTCAGAGCATTGATAGTTTTTTAC
ATTGGTACCAGCAGAAACCAGGGAAAGCCCCTAAGCTCCTGATCTATAGT
GCATCCGAGTTGCAAAGTGGGGTCCCATCACGTTTCAGTGGCAGTGGATC
TGGGACAGATTTCACTCTCACCATCAGCAGTCTGCATCCTGAAGATTTTG
CTACGTACTACTGTCAACAGGTTGTGTGGCGTCCTTTTACGTTCGGCCAA
GGGACCAAGGTGGAAATCAAACGG

### TAR1-5-22 (SEQ ID NO: 118)

GACATCCAGATGACCCAGTCTCCATCCTCCCTGTCTGCATCTGTAGGAGA
CCGTGTCACCATCACTTGCCGGGCAAGTCAGAGCATTGATTCTTATTTAC
ATTGGTACCAGCAGAAACCAGGGAAAGCCCCTAAGCTCCTGATCTATAGT
GCATCCCTGTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

### TAR1-5-23 (SEQ ID NO: 119)

GACATCCAGATGACCCAGTCTCCATCCTCCCTGTCTGCATCTGTAGGAGA
CCGTGTCACCATCACTTGCCGGGCAAGTCAGAGCATTGATCAGTATTTAC
ATTGGTACCAGCAGAAACCAGGGAAAGCCCCTAAGCTCCTGATCTATTCT
GCATCCCTTTTGCAAAGTGGGGTCCCATCACGTTTCAGTGGCAGTGGATC
TGGGACAGATTTCACTCTCACCATCAGCAGTCTGCAACCTGAAGATTTTG
CTACATACTACTGTCAACAGGTTGTGTGGCGTCCTTTTACGTTCGGCCAA
GGGACCAAGGTGGAAATCAAACGG

### TRR1-5-24 (SEQ ID NO: 120)

GACATCCAGATGACCCAGTCTCCATCCTCCCTGTCTGCATCTGTAGGAGA
CCGTGTCACCATCACTTGCCGGGCAAGTCAAAGCATTGATGAGTTTTTAC
ATTGGTACCAGCAGAAACCAGGGAAAGCCCCTAAGCTCCTGATCTATTGT
GCATCCCAGTTGCAAAGTGGGGTCCCATCACGTTTCAGTGGCAGTGGATC
TGGGACAGATTTCACTCTCACCATCAGCAGTCTACATCCTGAAGATTTTG
CTACGTACTACTGTCAACAGGTTGTGTGGCGTCCTTTTACGTTCGGCCAA
GGGACCAAGGTGGAAATCAAACGG

### TAR1-5-25 (SEQ ID NO: 121)

GACATCCAGATGACCCAGTCTCCATCCTCCCTGTCTGCATCTGTAGGAGA
CCGTGTCACCATCACTTGCCGGGCAAGTCAGAGCATTGATGCGTATTTAC
ATTGGTACCAGCAGAAACCAGGGAAAGCCCCTAAGCTCCTGATCTATTCT
GCATCCCTGTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

### TAR1-5-26 (SEQ ID NO: 122)

GACATCCAGATGACCCAGTCTCCATCCTCCCTGTCTGCATCTGTAGGAGA
CCGTGTCACCATCACTTGCCGGGCAAGTCAGAGCATTGATAGGTATTTAC
ATTGGTACCAGCAGAAACCAGGGAAAGCCCCTAAGCTCCTGATCTATAGT
GCATCCGTGTTGCAAAGTGGGGTCCCATCACGTTTCAGTGGCAGTGGATC
TGGGACAGATTTCACCCTCACCATCAGCAGTCTGCAGCCTGAAGATTTTG
CTACGTACTACTGTCAACAGGTTGTGTGGCGTCCTTTTACGTTCGGCCAA
GGGACCAAGGTGGAAATCAAACGG

### TAR1-5-27 (SEQ ID NO: 123)

GACATCCAGATGACCCAGTCTCCATCCTCCCTGTCTGCATCTGTAGGAGA
CCGTGTCACCATCACTTGCCGGGCAAGTCAGAGCATTGATAAGTATTTAC
ATTGGTACCAGCAGAAACCAGGGAAAGCCCCTAAGCTCCTGATCTATAGT
GCATCCTCGTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

### TAR1-5-28 (SEQ ID NO: 124)

GACATCCAGATGACCCAGTCTCCATCCTCCCTGTCTGCATCTGTAGGAGA
CCGTGTCACCATCACTTGCCGGGCAAGTCAGAGCATTGATCATTATTTAC
ATTGGTACCAGCAGAAACCAGGGAAAGCCCCTAAGCTCCTGATCTATAGT
GCATCCGTTTTGCAAAGTGGGGTCCCATCACGTTTCAGTGGCAGTGGATC
TGGGACAGATTTCACTCTCACCATCAGCAGTCTGCAACCTGAAGATTTTG
CAACGTACTACTGTCAACAGGTTGTGTGGCGTCCTTTTACGTTCGGCCAA
GGGACCAAGGTGGAAATCAAACGG

### TAR1-5-29 (SEQ ID NO: 125)

GACATCCAGATGACCCAGTCTCCATCCTCCCTGTCTGCATCTGTAGGAGA

CCGTGTCACCATCACTTGCCGGGCAAGTCAGAGCATTGATGAGTTTTTAC
ATTGGTACCAGCAGAAACCAGGGAAAGCCCCTAAGCTCCTGATCTATAGT
GCATCCATTTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

### TAR1-5-34 (SEQ ID NO: 126)

GACATCCAGATGACCCAGTCTCCATCCTCCCTGTCTGCATCTGTAGGAGA
CCGTGTCACCATCACTTGCCGGGCAAGTCAGAGCATTCAGACTGCGTTAC
TGTGGTACCAGCAGAAACCAGGGAAAGCCCCTAAGCTCCTGATCTATAAT
GCATCCAGTTTGCAAAGTGGGGTCCCATCACGTTTCAGTGGCAGTGGATC
TGGGACAGATTTCACTCTCACCATCAGCAGTCTGCAACCTGAAGATTTTG
CTACATACTACTGTCAACAGGTTGTGTGGCGTCCTTTTACGTTCGGCCAA
GGGACCAAGGTGGAAATCAAACGG

### TAR1-5-35 (SEQ ID NO: 127)

GACATCCAGATGACCCAGTCTCCATCCTCCCTGTCTGCATCTGTAGGAGA
CCGTGTCACCATCACTTGCCGGGCAAGTCAGAGCATTGATCAGTATTTAC
ATTGGTACCAGCAGAAACCAGGGAAAGCCCCTAAGCTCCTGATCTATGGT
GCATCCAATTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

### TAR1-5-36 (SEQ ID NO: 128)

GACATCCAGATGACCCAGTCTCCATCCTCCCTGTCTGCATCTGTAGGAGA
CCGTGTCACCATCACTTGCCGGGCAAGTCAGAGCATTGATAATTATTTAC
ATTGGTACCAGCAGAAACCAGGGAAAGCCCCTAAGCTCCTGATCTATAGT
GCATCCCAGTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

### TAR1-5-464 (SEQ ID NO: 129)

GACATCCAGATGACCCAGTCTCCATCCTCCCTGTCTGCATCTGTAGGAGA
CCGTGTCACCATCACTTGCCGGGCAAGTCAGAGCATTGATAATTTTTTAC

ATTGGTACCAGCAGAAACCAGGGAAAGCCCCTAAGCTCCTGATCTATAGT
GCATCCGAGTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

### TAR1-5-463 (SEQ ID NO: 130)

GACATCCAGATGACCCAGTCTCCATCCTCCCTGTCTGCATCTGTAGGAGA
CCGTGTCACCATCACTTGCCGGGCAAGTCAGAGCATTGATGAGTATTTAC
ATTGGTACCAGCAGAAACCAGGGAAACCCCCTAAGCTCCTGATCTATTCT
GCATCCAGTTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

### TAR1-5-460 (SEQ ID NO: 131)

GACATCCAGATGACCCAGTCTCCATCCTCCCTGTCTGCATCTGTAGGAGA
CCGTGTCACCATCACTTGCCGGGCAAGTCAGAGCATTGATCATTTTTTAC
ATTGGTACCAGCAGAAACCAGGGAAAGCCCCTAAGCTCCTGATCTATAGT
GCATCCGAGTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

### TAR1-5-461 (SEQ ID NO: 132)

GACATCCAGATGACCCAGTCTCCATCCTCCCTGTCTGCATCTGTAGGAGA
CCGTGTCACCATCACTTGCCGGGCAAGTCAGAGCATTGATAATTATTTAC
ATTGGTACCAGCAGAAACCAGGGAAAGCCCCTAAGCTCCTGATCTATTCG
GCATCCATGTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

### TAR1-5-479 (SEQ ID NO: 133)

GACATCCAGATGACCCAGTCTCCATCCTCCCTGTCTGCATCTGTAGGAGA
CCGTGTCACCATCACTTGCCGGGCAAGTCAGAGCATTGATGAGTATTTAC
ATTGGTACCAGCAGAAACCAGGGAAAGCCCCCAAGCTCCTGATCTATTCT

GCATCCATTTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

### TAR1-5-977 (SEQ ID NO: 134)

GACATCCAGATGACCCAGTCTCCATCCTCCCTGTCTGCATCTGTAGGAGA
CCGTGTCACCATCACTTGCCGGGCAAGTCAGAGCATTGATGAGTTTTTAC
ATTGGTACCAGCAGAAACCAGGGAAAGCCCCTAAGCTCCTGATCTATTCG
GCATCCGCTTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

### TAR1-5-478 (SEQ ID NO: 135)

GACATCCAGATGACCCAGTCTCCATCCTCCCTGTCTGCATCTGTAGGAGA
CCGTGTCACCATCACTTGCCGGGCAAGTCAGAGCATTGATGAGTATTTAC
ATTGGTACCAGCAGAAACCAGGGAAAGCCCCTAAGCTCCTGATCTATTCT
GCATCCATTTTGCAAAGTGGGGTCCCATCACGTTTCAGTGGCAGTGGATC
TGGGACAGATTTCACTCTCACCATCAGCAGTCTGCACCCTGAAGATTTTG
CTACGTACTACTGTCAACAGGTTGTGTGGCGTCCTTTTACGTTCGGCCAA
GGGACCAAGGTGGAAATCAAACGG

### TAR1-5-476 (SEQ ID NO: 136)

GACATCCAGATGACCCAGTCTCCATCCTCCCTGTCTGCATCTGTAGGAGA
CCGTGTCACCATCACTTGCCGGGCAAGTCAGAGCATTGATAATTATTTAC
ATTGGTACCAGCAGAAACCAGGGAAAGCCCCTAAGCTCCTGATCTATGCT
GCATCCAGTTTGCAAAGTGGGGTCCCATCACGTTTCAGTGGCAGTGGATC
TGGGACAGATTTCACTCTCACCATCAGCAGTCTGCAACCTGATGATTTTG
CTACGTACTACTGTCAACAGGTTGTGTGGCGTCCTTTTGCGTTCGGCCAA
GGGACCAAGGTGGAAATCAAACGG

### TAR1-5-490 (SEQ ID NO: 137)

GACATCCAGATGACCCAGTCTCCATCCTCCCTGTCTGCATCTGTAGGAGA
CCGTGTCACCATCACTTGCCGGGCAAGTCAGAGCATTGATAGTTATTTAC
ATTGGTACCAGCAGAAACCAGGGAAAGCCCCTAAGCTCCTGATCTATAGT
GCATCAAATTTAGAAACAGGGGTCCCATCACGTTTCAGTGGCAGTGGATC

TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

### TAR1h-1 (SEQ ID NO: 138)

GACATCC AGATGACCCAGTCTCCATCCTCCCTGTCTGCATCTGTAGGAGA
CCGTGTCACCATCACTTGCCGGGCAAGTCAGGTGATTTGGATGCGTTAG
ATTGGTACCAGCAGAAACCAGGGAAAGCCCCTAAGCTCCTGATCTATAGT
GCGTCCCGTTTGCAAAGTGGGGTCCCATCACGTTTCAGTGGCAGTGGATC
TGGGACAGATTTCACTCTCACCATCAGCAGTCTGCATCCTGAAGATTTTG
CTACGTACTACTGTCAACAGTATGCTGTGTTTCCTGTGACGTTCGGCCAA
GGGACCAAGGTGGAAATCAAACGG

### TAR1h-2 (SEQ ID NO: 139)

GACATCCAGATGACCCAGTCTCCATCCTCCCTGTCTGCATCTGTAGGAGA
CCGTGTCACCATCACTTGCCGGGCAAGTCAGACTATTTATGATGCGTTAA
GTTGGTACCAGCAGAAACCAGGGAAAGCCCCTAAGCTCCTGATCTATGGT
GGTTCCAGGTTGCAAAGTGGGGTCCCATCACGTTTCAGTGGCGGTGGATC
TGGGACAGATTTCACTCTCACCATCAGCAGTCTGCAACCTGAAGATTTTG
CTACGTACTACTGTCAACAGTATAAGACTAAGCCTTTGACGTTCGGCCAA
GGGACCAAGGTGGAAATCAAACGG

### TAR1h-3 (SEQ ID NO: 140)

GACATCCAGATGACCCAGTCCCCATCCTCCCTGTCTGCATCTGTAGGAGA
CCGTGTCACCATCACTTGCCGGGCAAGTCAGACTATTTATGATGCGTTAA
GTTGGTACCAGCAGAAACCAGGGAAAGCCCCTAAGCTCCTGATCTATGGT
GGTTCCAGGTTGCAAAGTGGGGTCCCATCACGTTTCAGTGGTAGTGGATC
TGGGACAGATTTCACTCTCACCATCAGCAGTCTGCAACCCGAAGATTTTG
CTACGTACTACTGTCAACAGTATGCTCGTTATCCTCTTACGTTCGGCCAA
GGGACCAAGGTGGAAATCAAACGG

The sequence of the TAR1-5-19 anti-human TNP-α dAb adapted to various formats in these examples is as follows:

### TAR1-5-19

### Amino acid (SEQ ID NO: 191)

DIQMTQSPSSLSASVGDRVTITCRASQSVKEFLWWYQQKPGKAPKLLIYMASNLQSGVPS
RFSGSGSGTDFTLTISSLQPEDFATYYCQQKFKLPRTFGQGTKVEIKR

### Nucleotide (SEQ ID NO: 115)

gacatccagatgacccagtctccatcctccctgtctgcatctgtaggagaccgtgtcacc
atcacttgccgggcaagtcagagcgttaaggagtttttatggtggtaccagcagaaacca
gggaaagcccctaagctcctgatctatatggcatccaatttgcaaagtggggtcccatca
cgtttcagtggcagtggatctgggacagatttcactctcaccatcagcagtctgcaacct
gaagattttgctacgtactactgtcaacagaagtttaagctgcctcgtacgttcggccaa
gggaccaaggtggaaatcaaacgg

### Example 2: Efficacy study of PEGylated TAR1-5-19 in a prophylactic model of arthritis.

Tg197 mice are transgenic for the human TNF-globin hybrid gene and heterozygotes at 4-7 weeks of age develop a chronic, progressive polyarthritis with histological features in common with rheumatoid arthritis [Keffer, J., Probert, L.,Cazlaris, H., Georgopoulos, S.,Kaslaris, E., Kioussis, D., Kollias, G. (1991). Transgenic mice expressing human tumor necrosis factor: a predictive genetic model of arthritis. EMBO J., Vol. 10, pp. 4025-4031.]

To test the efficacy of a PEGylated dAb (PEG format being 2x20k branched with 2 sites for attachment of the dAb [i.e. 40K mPEG2 MAL2], the dAb being TAR1-5-19cys) in the prevention of arthritis in the Tg197 model, heterozygous transgenic mice were divided into groups of 10 animals with equal numbers of male and females. Treatment commenced at 3 weeks of age with weekly intraperitoneal injections of test items. The expression and PEGylation of TAR1-5-19cys monomer is outlined in Section 1.3.3, example 1. All protein preparations were in phosphate buffered saline and were tested for acceptable levels of endotoxins.

The study was performed blind. Each week the animals were weighed and the macrophenotypic signs of arthritis scored according to the following system: 0 = no arthritis (normal appearance and flexion), 1 = mild arthritis (joint distortion), 2 = moderate arthritis (swelling, joint deformation), 3 = heavy arthritis (severely impaired movement).

The outcome of the study clearly demonstrated that 10mg/kg PEGylated TAR1-5-19 inhibited the development of arthritis with a significant difference between the arthritic scoring of the saline control and treated group. The 1mg/kg dose of PEGylated TAR1-5-19 also produced a statistically significantly lower median arthritic score than saline control group (P<0.05% using normal approximation to the Wilcoxon Test).

### Example 3: Efficacy study of PEGylated TAR1-5-19 in a therapeutic model of arthritis.

To test the efficacy of a PEGylated dAb in the therapeutic model of arthritis in the Tg197 model, heterozygous transgenic mice were divided into groups of 10 animals with equal numbers of male and females. Treatment commenced at 6 weeks of age when the animals had significant arthritic phenotypes. Treatment was twice weekly with 4.6mg/kg intraperitoneal injections of test items. The sample preparation and disease scoring are as described above in Example 2.

The arthritic scoring clearly demonstrated that PEGylated TAR1-5-19 inhibited the progression of arthritis in a therapeutic model, The 4.6mg/kg dose of PEGylated TAR1-5-19 produced a statistically significantly lower median arthritic score than saline control group at week 9 (P<0.01% using normal approximation to the Wilcoxon Test).

### Example 4: dAb Efficacy in a Slow Release Format

To test the efficacy of a dAb from a slow release format, a dAb with a small PEG molecule (where the PEG is 4x5k with four sites for attachment of a dAb with a C-terminal cys residue, the dAb being TAR 1-5-19 [i.e. 20K PEG 4 arm MAL]) was loaded into a 0.2 ml osmotic pump. The pump had a release rate of 0.2 ml over a 4 week period was implanted subcutaneously into mice at week 6 in the therapeutic Tg197 model as described above. The arthritic scores of these animals increased at a clearly slower rate when compared to animals implanted with pumps loaded with saline. This demonstrates that dAbs are efficacious when delivered from a slow release format.

### Example 5: Half-life stabilized anti-human TNF-α dAb prevents the onset of RA in the Tg197 mouse model.

The dAb monomer TAR1-5-19 described herein is an affinity matured dAb monomer derived from a dAb initially selected using passively coated TNF-α. The initial clone had a ND50 in the L929 TNF-cytotoxicity neutralization assay greater than 5 µM. TAR1-5-19 has an ND50 of less than 30 nM. When formatted as an Fc Fusion as described herein, the TAR1-5-19 clone has an ND50 of less than 5 nM in the L929 assay.

The serum half-life of TAR1-5-19 dAb Fc-fusion was examined following injection into mice. Where the TAR 1-5-19 dAb monomer had a t1/2β of approximately 20 minutes, the Fc-fusion formatted version of the same dAb had a t1/2β of greater than 24 hours, representing a greater than 70-fold increase in serum half-life.

The TAR1-5-19 dAb Fc fusion construct was tested in the Tg197 mouse model of RA described herein above. Mice were divided into five groups of 10, with equal numbers of male and female mice per group. Treatment with twice weekly IP injections of TAR1-5-19 dAb Fc fusion, ENBREL or saline was begun at 3 weeks of age, a time at which RA symptoms have not yet manifested. The study was conducted for 7 weeks. Two dosages of the TAR1-5-19 dAb Fc fusion, 1 mg/kg and 10 mg/kg, were administered. Negative control animals received a negative control anti-β-gal Fc fusion twice weekly at 10 mg/kg, and one group was treated twice weekly with saline injection. For comparison, one group received 10 ing/kg of ENBREL twice weekly.

Animals were assessed for arthritic scores as described herein, in a blinded manner. At the end of the 7 week course of treatment, animals receiving the twice weekly dosage of 10 mg/kg of the TAR1-5-19 dAb Fc fusion had lower arthritic scores than the animals receiving ENBREL at 10 mg/kg, and had experienced essentially complete prevention of arthritic disease relative to non-treated animals or animals receiving the negative control dAb Fc fusion.

TNF-α is associated with cachexia. Animals were weighed throughout the course of anti-TNF-α dAb treatment. The weights of the animals receiving the TAR1-5-19 dAb Fc fusion were significantly greater than those receiving negative control dAb Fc fusion and no treatment and similar to the weights of the animals receiving ENBREL injections.

In summary, 10 mg/kg TAR1-5-19 completely prevented the onset of arthritis in the Tg197 model. This response was dose-dependent, with a partial effect resulting from a 1 mg/kg dose, and the response was superior to that observed with a similar dose of the existing anti-TNF-α drug ENDREL. This study demonstrates the efficacy of dAbs as therapeutics in a clinically accepted model of human disease.

Histopathological analyses of fixed sections from the joints of the animals are in agreement with these data (not shown).

All publications, patents and published patent applications mentioned in the present specification, and references cited in said publications, are herein incorporated by reference. Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

## Claims

1. A single domain antibody polypeptide construct which comprises the amino acid sequence

DIQMTQSPSSLSASVGDRVTITCRASQSIDSYLHWYQQKPGKAPKLLIYSASN
LETGVPSRFSGSGSGTDFTLTISSLQPEDFATYYCQQVVWRPFTFGQGTKVEI
KR (TAR 1-5-490).

2. Use of a composition comprising a single domain antibody polypeptide construct that antagonizes human TNFα's binding to a receptor in vitro for the preparation of a medicament for the treatment, prevention, inhibition of progression or delay in the onset of rheumatoid arthritis, wherein the single domain antibody polypeptide comprises the sequence

DIQMTQSPSSLSASVGDRVTITCRASQSIDSYLHWYQQKPGKAPKLLIYSASN
LETGVPSRFSGSGSGTDFTLTISSLQPEDFATYYCQQVVWRPFTFGQGTKVEI
KR (TAR 1-5-490).

3. A method of treating rheumatoid arthritis, the method comprising administering to an individual in need thereof a therapeutically effective amount of a composition comprising a single domain antibody polypeptide construct that antagonizes human TNFα's binding to a receptor, whereby said rheumatoid arthritis is treated, wherein the single domain antibody polypeptide comprises the sequence

DIQMTQSPSSLSASVGDRVTITCRASQSIDSYLHWYQQKPGKAPKLLIYSASN
LETGVPSRFSGSGSGTDFTLTISSLQPEDFATYYCQQVVWRPFTFGQGTKVEI
KR (TAR 1-5-490).

4. A composition comprising a single domain antibody polypeptide construct that antagonizes human TNFα's binding to a receptor in vitro for use in the treatment, prevention, inhibition of progression or delay in the onset of rheumatoid arthritis, wherein the single domain antibody polypeptide comprises the sequence
